# EUROPEAN PATENT APPLICATION

(11) **EP 4 418 278 A1**
(43) Date of publication of application: **21.08.2024**
(21) Application number: 22881399.4
(22) Date of filing: 14.10.2022
(51) Int. Cl.: G16H 30/40, G16H 50/70, G16H 50/50, G16H 50/20, G06T 7/00, A61B 5/00, G06N 20/00

(54) **SKIN DIAGNOSIS SYSTEM AND METHOD BASED ON IMAGE ANALYSIS USING DEEP LEARNING**

(30) Priority: 14.10.2021 KR 20210136568; 13.10.2022 KR 20220131817
(71) Applicant: Amorepacific Corporation, Seoul 04386 (KR)
(72) Inventor: PARK, Hyeokgon, Yongin-si, Gyeonggi-do 17074 (KR); LEE, Sangran, Yongin-si, Gyeonggi-do 17074 (KR); HWANG, Joongwon, Yongin-si, Gyeonggi-do 17074 (KR); KIM, Eun Joo, Yongin-si, Gyeonggi-do 17074 (KR); OH, Wangjin, Yongin-si, Gyeonggi-do 17074 (KR)
(74) Representative: Viering, Jentschura & Partner mbB Patent- und Rechtsanwälte
(86) International application number: PCT/KR2022/015586
(87) International publication number: WO 2023/063772

(57) **Abstract**

Embodiments relate to a skin diagnosis system and method based on image analysis using deep learning, comprising: a face recognition model that derives shape or location information of a facial structure by recognizing feature points capable of identifying an individual in an acquired face image; a de-identification model that de-identifies a face image on the basis of the shape or location information of the facial structure such that personal information of an analysis target cannot be identified; and a plurality of artificial neural network models for each of at least one item among diagnoses for wrinkles, pigmentation, pores, erythema, and aging.

## Description

### [Technical Field]

The present application relates to a skin diagnosis system and method based on image analysis using deep learning.

### Cross Reference To Related Application

The present application claims the priorities of Korean Patent Application No. 10-2021-0136568 filed on October 14, 2021, and Korean Patent Application No. 10-2022-0131817 filed on October 13, 2022, the entire contents of which is incorporated herein by reference.

### [Background Art]

In addition to medical use, skin diagnosis is actively used in the cosmetics industry and related research fields. With the development of online markets and personalized services, a demand for methods to perform the skin diagnosis more easily and accurately is increasing. Meanwhile, various existing skin diagnosis and measurement methods have high accuracy, but have limited measurement items and require expensive equipment. Existing image analysis-based skin diagnosis methods are relatively inexpensive and can measure various items with a single image, but have low accuracy and are difficult or expensive to obtain detailed quantitative data.

Further, in order to avoid the difficulty of achieving accuracy and data labeling issues, the existing skin diagnosis methods have not provided detection location and quantitative data for symptoms or have realized the symptoms into simple grading judgments, which caused a problem of failing to provide actual symptom location and quantitative information for each item.

Furthermore, since the existing prior technologies do not include a procedure for removing personal identification information contained in a face image that is used for the skin diagnosis, there is a problem in that management or protection of the personal information is insufficient during analysis processing or data collection.

Other prior technologies only define a concept of the skin diagnosis using an image by artificial intelligence (AI), and do not include technical definitions for actual detailed operation or diagnosis, or limit image collection and diagnosis equipment to a smartphone, a smart device, etc., whereby the scope of applying the prior technologies is limited because they do not take into account a possible combination of collection environment and separate diagnostic service structure in various imaging devices.

### [Disclosure]

### [Technical Problem]

The present application is intended to solve the above-mentioned problems and provides a diagnostic technology capable of recognition and integrated analysis, such as facial skin aging, by applying artificial intelligence (AI) technology to skin aging evaluation and skin lesion evaluation. Specifically, by creating an artificial neural network model through deep learning, symptoms according to each item in the skin image can be diagnosed and analyzed to quantitatively derive detailed location for each subject, shape, and degree of severity of actual skin symptoms. It is possible to analyze the number, average size or shape of each symptom, the overall area or intensity of the symptom, etc., and the obtained information can be used to provide visualization information that emphasizes the diagnosis in the skin image.

### [Technical Solution]

An image analysis-based skin diagnosis method using deep learning performed by a processor according to an aspect of the present application comprises the steps of: acquiring a face image of a subject by photographing a target skin; deriving shape or location information of a facial structure by recognizing feature points capable of identifying an individual in the acquired face image; de-identifying the face image on the basis of the shape or location information of the facial structure such that personal information of an analysis target cannot be identified; and visualizing and providing skin diagnosis results for items corresponding to artificial neural network models and symptom locations for each item by inputting the de-identified face images into a plurality of the artificial neural network models , respectively, wherein the items may include at least one of diagnoses for wrinkles, pigmentation, pores, erythema, and aging.

In an embodiment, the step of de-identifying the face image may include: separating feature points and a background from the face image and removing a skin shape having the feature points; dividing the face image from which the feature points have been removed into a plurality of patches; and rearranging the plurality of patches by randomizing them.

In an embodiment, the feature points may be at least one of eyebrows, eyes, nose, and lips.

In an embodiment, each of the plurality of artificial neural network models is learned using a plurality of training samples as learning data, and the plurality of training samples may include transformations using a data augmentation technique.

In an embodiment, the data augmentation technique may include at least one of random crop, blur, and flip processing.

In an embodiment, the artificial neural network model for diagnosing the wrinkles may be learned by adjusting parameters including the total number of a subject with detected wrinkles, an estimate of intensity (depth) compared to the surrounding undetected area in the subject with detected wrinkles, the total area with detected wrinkles, the length and width of the detected wrinkles, and may output analysis results for at least one among the number of wrinkles, intensity (depth) of wrinkles, wrinkle area, wrinkle length, wrinkle width, distribution for each intensity, area, length or width of wrinkles, and wrinkle score.

In an embodiment, the artificial neural network model for diagnosing the pigmentation may be learned by adjusting parameters including the total number of a subject with detected pigmentation, an estimate of intensity compared to the surrounding undetected area in the subject with detected pigmentation, the total area with detected pigmentation, and may output analysis results for at least one among the number of pigmentation, intensity of pigmentation, pigmentation area, distribution for each intensity, area, length or width of pigmentation, and pigmentation score.

In an embodiment, the artificial neural network model for diagnosing the pores may be learned by adjusting parameters including the total number of a subject with detected pores, an estimate of intensity (depth) compared to the surrounding undetected area in the subject with detected pores, the total area with detected pores, pore length and pore width, and may output analysis results for at least one among the number of pores, intensity (depth) of pores, pore size, pore area, pore length, pore width, pore sagging (length to width ratio), distribution for each intensity, area, length, width or sagging of pores, and pore score.

In an embodiment, the artificial neural network model for diagnosing the erythema may be learned by adjusting parameters including the total number of a subject with detected erythema, an estimate of intensity compared to the surrounding undetected area in the subject with detected erythema, the total area with detected erythema, and may output analysis results for at least one among the number of erythema, intensity of erythema, erythema area, distribution for each intensity or area of erythema, and erythema score.

In an embodiment, the artificial neural network model for diagnosing the aging may predict age for facial aging or facial skin aging estimated from a face image by inputting at least one of the de-identified face image, the output result of a single artificial neural network model, and the value that integrates the output result of a plurality of artificial neural network models.

In an embodiment, each of the plurality of artificial neural network models may be an encoder-decoder structural model based on U-net model.

In an embodiment, each of the plurality of artificial neural network models may be learned in the form of an imageNet pre-trained weight based on ResNet.

In an embodiment, the present method may further comprise the step of evaluating at least one of antioxidant efficacy and whitening efficacy for a specific product based on skin diagnosis results for the above items.

In an embodiment, the present method may further comprise the step of, before inputting the de-identified face image into the plurality of artificial neural network models, obtaining information about the subject's skin concerns and lifestyle through a questionnaire, wherein the result of skin diagnose is the one resulting from the subject's skin concerns and lifestyle.

In an embodiment, the present method may further comprise the step of recommending a specific product tailored to the subject's skin concerns and lifestyle or providing beauty eating habit, based on the result of skin diagnose.

According to another aspect of the present application, a skin diagnosis system based on image analysis using deep learning comprises: an imaging unit that acquires a face image by photographing a target skin; a face detection model that derives shape or location information of a facial structure by recognizing feature points capable of identifying an individual in the acquired face image; a de-identification model that de-identifies the face image on the basis of the shape or location information of the facial structure such that personal information of an analysis target cannot be identified; and a plurality of artificial neural network models for each of at least one item among diagnoses for wrinkles, pigmentation, pores, erythema, and aging, wherein the plurality of artificial neural network models receive the de-identified face image as input, and visualize and provide skin diagnosis results for items corresponding to the artificial neural network models and symptom locations for each item.

### [Advantage Effects]

A skin diagnosis system and method based on image analysis using deep learning according to an aspect of the present application are separately composed of a face detection model that finds location of a face, a de-identification model that leaves only the skin area in a face image and performing random arrangement to de-identify personal information, and a model for each diagnostic symptom including diagnoses for wrinkles, pigmentation, pores, erythema, and aging. Therefore, optimization and function separation or adjustment can be performed for each characteristic, which enables overlapping diagnosis and efficient system management and operation.

Skin diagnosis can be performed even with a partial skin image that has been de-identified from the acquired face image. Since the diagnosis system is designed not to utilize, store, and/or process personal information, system operation and data collection can proactively respond to management without leaking the personal information.

The images used in the system to diagnose skin condition are not limited to images captured from a professional photography equipment, a general camera, or a smartphone, and are designed to derive the characteristics of each skin diagnosis item according to the image condition so that the diagnosis system can be used for various purposes by minimizing the impact on a type or form of the image.

In long-term research on skin aging, such as anti-aging research, that requires long-term follow-up analysis, and evaluation of the efficacy of anti-aging products, and evaluation of the efficacy of whitening cosmetics that require analysis of subtle change in skin pigment, evaluation of skin change and the efficacy of cosmetics can be quantitatively performed. Therefore, various skin diagnostic items can be performed quickly and at low cost without a separate skin measurement equipment or analysis preparation, thereby providing advantageous characteristics in the fields such as long-term follow-up or large-scale skin research.

The effects of the present application are not limited to the effects mentioned above, and other effects not mentioned will be clearly understood by those skilled in the art from the description of the claims.

### [Description of the Drawings]

In order to disclose the technical solutions of the embodiments of the present application or the prior art more clearly, drawings necessary to describe the embodiments are briefly introduced below. It should be understood that the drawings below are intended only to illustrate the embodiments of the present specification and are not intended to limit them. Also, for clarity of explanation, some elements to which various variations such as exaggeration and omission are applied may be illustrated in the drawings below.
FIG. 1 is a block diagram of an image analysis-based skin diagnosis system according to an embodiment of the present application.
FIG. 2 is a flowchart of an image analysis-based skin diagnosis method according to another embodiment of the present application.
FIG. 3 is a schematic diagram showing an input/output process of a plurality of artificial neural network models according to an embodiment of the present application.
FIG. 4 shows a process in which a face detection model according to an embodiment of the present application extracts feature points of a face to locate them, and extracts only the skin area through a de-identification model.
FIG. 5 shows a de-identification process of a face image of a de-identification model according to an embodiment of the present application.
FIGS. 6A to 6D illustrate a process in which a plurality of training samples are labeled by an expert in order to learn each of a plurality of artificial intelligence neural network models according to an embodiment of the present application.
FIG. 7 shows artificial intelligence neural network models based on U-net according to an embodiment of the present application.
FIG. 8 is a table that summarizes main items for diagnosing a skin by each of a plurality of artificial intelligence neural network models according to an embodiment of the present application.
FIGS. 9A to 9D are images in which the diagnosis results for each item of de-identification models and a plurality of artificial intelligence neural network models are reconstructed into images before de-identification according to an embodiment of the present application.
FIGS. 10A to 10D are diagnostic images for each item of a plurality of artificial intelligence neural network models when de-identification is performed using de-identification models according to an embodiment of the present application.
FIGS. 11A to 11D are graphs showing correlation between the diagnostic results for each item as age increases, according to an embodiment of the present application.
FIGS. 12A and 12B are graphs that analyze the results of an anti-aging product evaluation study according to an embodiment of the present application.
FIGS. 13A and 13B are graphs that analyze the results of a whitening product evaluation study according to an embodiment of the present application.
FIG. 14 shows a process for obtaining information about a subject's skin concerns and lifestyle through a questionnaire according to an embodiment of the present application.

### [Mode for Invention]

The terminology used herein is only intended to refer to specific embodiments and is not intended to limit the present application. As used herein, singular forms also include plural forms unless the phrases clearly indicate the contrary. As described in the specification, the meaning of "comprising" refers to specify a certain characteristic, area, integer, step, operation, item and/or component, and does not exclude the presence or addition of another characteristic, area, integer, step, operation, item and/or component.

Although not defined differently, all the terms including technical and scientific terms used herein have the same meaning as those generally understood by a person who has an ordinary knowledge in the technical field to which the present application belongs. The terms defined in commonly used dictionaries are further interpreted as having meanings consistent with related technical literature and currently disclosed contents, and are not interpreted in ideal or very formal meanings unless defined otherwise.

Hereinafter, embodiments of the present application will be reviewed in detail with reference to the drawings.

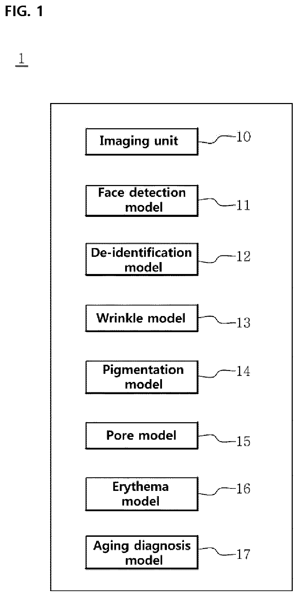

FIG. 1 is a block diagram of a skin diagnosis system 1 based on image analysis using deep learning (hereinafter referred to as "skin diagnosis system") according to an embodiment of the present application.

Referring to FIG. 1, the skin diagnosis system 1 may comprise: an imaging unit 10 that acquires a face image by photographing a target skin; a face detection model 11 that derives shape or location information of a facial structure by recognizing feature points capable of identifying an individual in the acquired face image; a de-identification model 12 that de-identifies the face image on the basis of the shape or location information of the facial structure such that personal information of an analysis target cannot be identified; and a plurality of artificial neural network models 13, 14, 15, 16 for each of at least one item among diagnoses for wrinkles, pigmentation, pores, and erythema, wherein the plurality of artificial neural network models receive the de-identified face image as input, and visualize and provide skin diagnosis results for items corresponding to the artificial neural network models and symptom locations for each item. The items may include at least one of wrinkles, pigmentation, pores, and erythema. Further, the skin diagnosis system 1 may further comprise an aging diagnosis model 17 that predicts age estimated from the face image by inputting the value that integrates the output results of a single artificial neural network model and a plurality of artificial neural network models including the de-identification model.

In this specification, the artificial neural network model may include a deep learning model, wherein the deep learning model may be in the form of artificial neural networks stacked in multiple layers. The deep learning model automatically learns the features of each image by learning a large amount of data in a deep neural network consisting of a network of the multiple layers, and through this, trains the network in the manner of minimizing errors in the objective function, that is, the prediction accuracy.

In this specification, the deep learning model may use, for example, CNN (Convolutional Neural Network), DHN (Deep Hierachical Network), CDBN (Convolutional Deep Belief Network), DDN (Deconvolutional Deep Network), etc., but a variety of deep learning models are available now or in the future.

The skin diagnosis system 1 according to embodiments may be entirely hardware, entirely software, or may have aspects that are partly hardware and partly software. For example, the system may collectively refer to a hardware equipped with data processing capabilities and an operating software for driving the hardware. In this specification, the terms such as "unit," "system," and "device" are intended to refer to a combination of a hardware and a software driven by the hardware. For example, the hardware may be a data processing device that includes CPU (Central Processing Unit), GPU (Graphics Processing Unit), or other processor. Further, the software may refer to an executing process, object, executable file, thread of execution, program, etc.

For example, the concepts (e.g., operation of the components 10 to 17) of the skin diagnosis system 1 (such as the VISIA-CR^{®} system from Canfield Company (Fairfield, NJ, USA)) may be integrated into various computing devices (e.g., a smart device including a smartphone, a computer, etc.) as an imaging system for skin analysis, a software for the computer, or a web for customer. The software may be designed to process captured images of a target skin, and may diagnose and evaluate the target skin in the images and further provide the operation results to the user.

The imaging unit 10 is a component that photographs the target skin, and may be implemented with various imaging devices that can be used in the present technical field. In an embodiment, the imaging unit 10 is configured to acquire a face image by photographing an image of the target skin. For example, the imaging unit 10 may be implemented with VISIA-CR^{®} from Canfield Company (Fairfield, NJ, USA).

The imaging unit 10 may provide image data of the target skin to other components 11 to 17. Additionally, the imaging unit 10 may be further configured to perform operation of extracting a partial area of the target skin's image, for example, a cropping processing operation.

It will be apparent to those skilled in the art that the skin diagnosis system 1 may comprise other components which are not explicitly described herein. For example, the skin diagnosis system may comprise other hardware elements necessary for the operations described herein, including a network interface, an input device for data entry, an output device for display, printing or other data presentation, and a storage device such as memory.

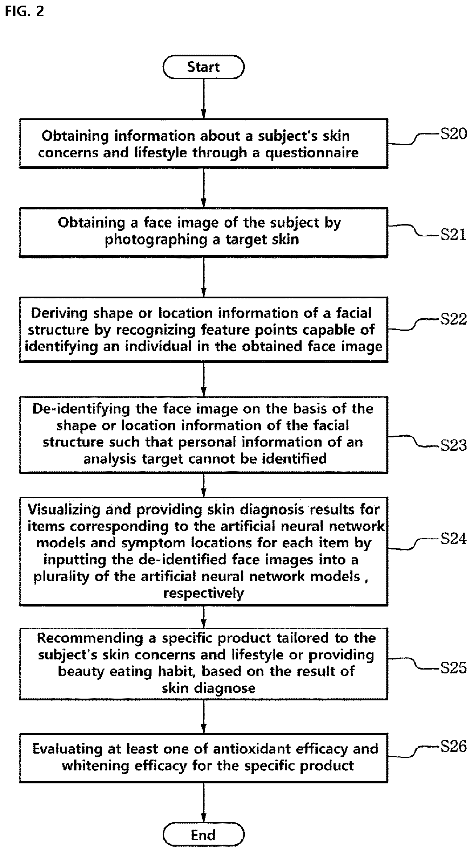

FIG. 2 is a flowchart of an image analysis-based skin diagnosis method according to another embodiment of the present application.

Referring to FIG. 2, the image analysis-based skin diagnosis method (hereinafter, "skin diagnosis method") is performed by a computing device including a processor. For example, the computing device including the processor may be performed by the skin diagnosis system 1 or some components (e.g., an imaging unit 10, a face detection model 11, a de-identification model 12, and/or a plurality of artificial neural network models 13 to 17), or may be performed by another computing device. Hereinafter, for clarity of explanation, the present application will be described in more detail with embodiments in which the skin diagnosis method is performed by the skin diagnosis system 1.

Referring to FIG. 2, the skin diagnosis method comprises the steps of: obtaining information about a subject's skin concerns and lifestyle through a questionnaire (S20); obtaining a face image of the subject by photographing a target skin (e.g., by the imaging unit 10) (S21); deriving shape or location information of a facial structure by recognizing feature points capable of identifying an individual in the obtained face image (e.g., by the face detection model 11) (S22); de-identifying the face image on the basis of the shape or location information of the facial structure such that personal information of an analysis target cannot be identified (e.g., by the de-identification model 112) (S23); visualizing and providing skin diagnosis results for items corresponding to the artificial neural network models and symptom locations for each item by inputting the de-identified face images into a plurality of the artificial neural network models , respectively (e.g., by the plurality of artificial neural network models 13 to 17) (S24); recommending a specific product tailored to the subject's skin concerns and lifestyle or providing beauty eating habit, based on the result of skin diagnose (S25); and evaluating at least one of antioxidant efficacy and whitening efficacy for the specific product (S26).

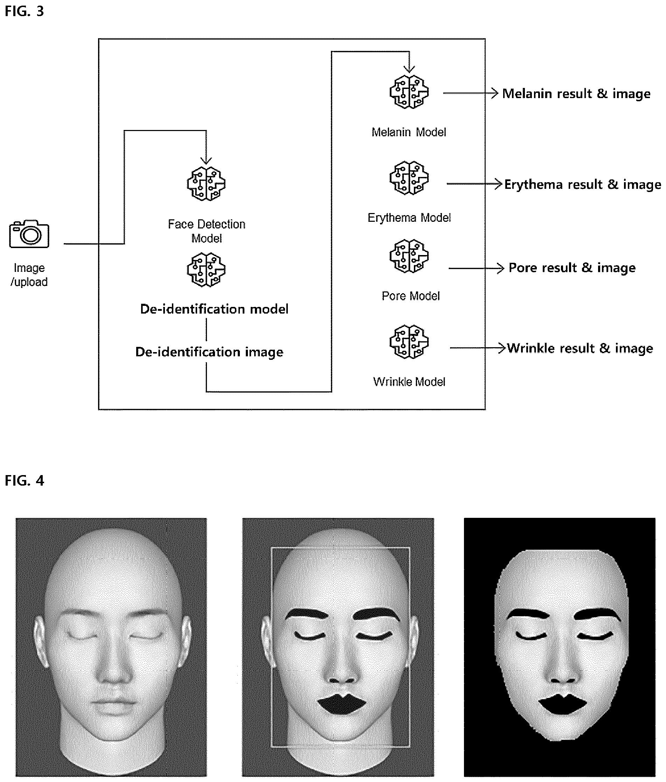

FIG. 3 is a schematic diagram showing an input/output process of a plurality of artificial neural network models according to an embodiment of the present application.

Referring to FIG. 3, the skin diagnosis system 1 consists of total 6 types of a face detection model that finds location of the face, a de-identification model that removes personal information from the image, diagnosis models for each item for diagnosing pigmentation, pores, erythema, and wrinkles, etc., thereby enabling optimization of each characteristic and separation of functions. Each model is trained to accurately diagnose symptoms for each item.

The wrinkle model that diagnoses wrinkles can output analysis results for at least one among the number of wrinkles, intensity (depth) of wrinkles, wrinkle area, wrinkle length, wrinkle width, distribution for each intensity, area, length or width of wrinkles, and wrinkle score, by inputting the de-identified face image. The pigmentation model that diagnoses pigmentation can output analysis results for at least one among the number of pigmentation, intensity of pigmentation, pigmentation area, distribution for each intensity or area of pigmentation, and pigmentation score. The pore model that diagnoses pores can output analysis results for at least one among the number of pores, intensity (depth) of pores, pore size, pore area, pore length, pore width, pore sagging (length to width ratio), distribution for each intensity, area, length, width or sagging of pores, and pore score. The erythema model that diagnoses erythema can output analysis results for at least one among the number of erythema, intensity of erythema, erythema area, distribution for each intensity or area of erythema, and erythema score. Each of the wrinkle score, the pigmentation score, the pore score, and the erythema score means a scored numeral value for the skin condition of each individual skin item based on pre-collected data, and is calculated by considering single or multiple combinations of data such as the intensity and total area of wrinkles, pigmentation, pores, and erythema, respectively. The analysis results output from the wrinkle model, pigmentation model, pore model, and erythema model can be visually displayed in the area corresponding to the symptom of the item. In addition, the aging diagnosis model that diagnoses facial aging can output analysis results on the facial aging and the facial skin aging by inputting the de-identified or identified face images.

FIG. 4 shows a process in which a face detection model according to an embodiment of the present application extracts feature points of a face to locate them, and extracts only the skin area through a de-identification model.

Referring to FIG. 4, the face detection model can derive shape or location information of a facial structure by recognizing feature points capable of identifying an individual in an acquired face image. The feature points may be at least one of eyebrows, eyes, nose, and lips, and may be recognized separately from the background. In an embodiment, the face detection model may determine whether the image is suitable for skin diagnosis and proceed with skin analysis only if it is determined to be the suitable image. Additionally, the face detection model may output a facial size, which is the size of the detected skin area, after de-identifying the face image.

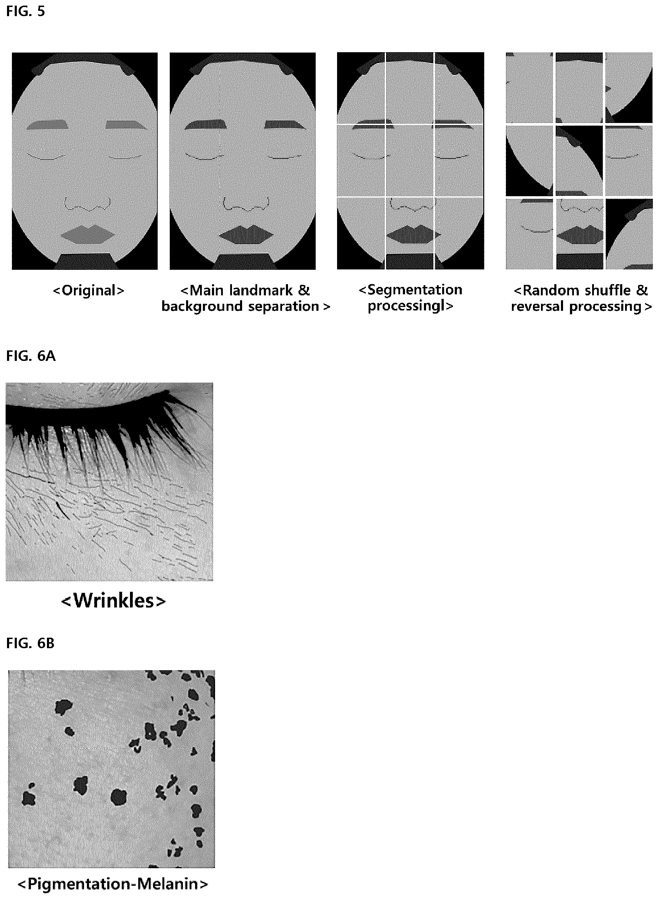

FIG. 5 shows a de-identification process of a face image of a de-identification model according to an embodiment of the present application.

Referring to FIG. 5, the de-identification model may include the steps of separating feature points and a background from the face image and removing a skin shape having the feature points; dividing the face image from which the feature points have been removed into a plurality of patches; and rearranging the plurality of patches by randomizing them.

Therefore, the skin diagnosis can be performed even with a partial skin image that has been de-identified from the acquired face image through the de-identification model. Since the diagnosis system is designed not to utilize, store, and/or process personal information, system operation and data collection can proactively respond to management without leaking the personal information.

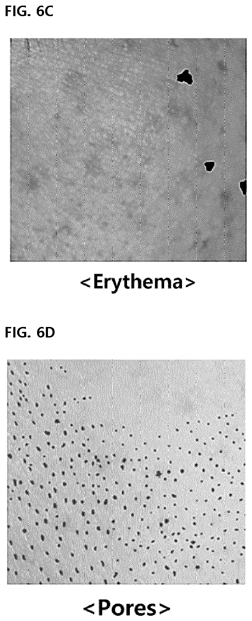

FIGS. 6A to 6D illustrate a process in which a plurality of training samples are labeled by an expert in order to learn each of a plurality of artificial intelligence neural network models according to an embodiment of the present application.

Referring to FIGS. 6A to 6D, each of the plurality of artificial neural network models can be learned using the plurality of training samples as learning data. In an experimental example, 13,596 images for Korean men and women's research on human body application taken with a full-eye imaging device (VISIA-CR, VISIA, etc.) of a high-resolution were selected and processed, 50,358 image patches were used to learn the artificial neural network model, and 1,150 image patches were used to verify model learning results. In case of data labeling, a location and shape of each item capable of identifying from the skin image with the naked eye were segmented, and the worked data was inspected and produced by workers of 50 or more and clinical experts of 3 or more having experience of 10 years or more. The data labeling is a process of displaying final results or information on original data so that the artificial intelligence can learn according to the development goal. For example, in order to learn wrinkle diagnosis, a wrinkle model displays exact location of the wrinkles on the skin image to be diagnosed. The segmentation is a work that classifies and displays a type and location of a specific target area when labeling the image data or analyzing the images.

The plurality of training samples may be modified using data augmentation technique. The data augmentation is a technique that increases adaptability to various data environments and development performance by transforming data sets for the artificial intelligence learning into various realistic shapes to secure data diversity and quantity beyond the actual collected data.

In an embodiment, the data augmentation technique may include at least one of random crop, blur, and flip processing. Through the data augmentation technique, general and consistent results can be obtained even in various environments, thereby being capable of exerting the effect of enabling meaningful operation even in untrained devices or environments.

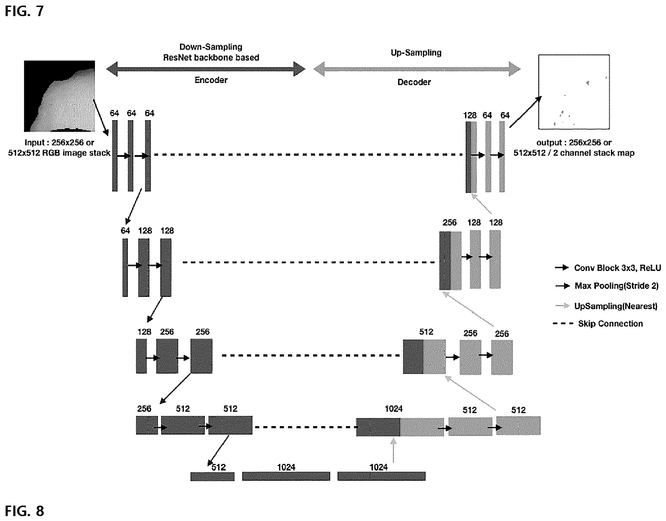

FIG. 7 shows artificial intelligence neural network models based on U-net according to an embodiment of the present application.

Referring to FIG. 7, each of the de-identification model and the plurality of artificial neural network models may be an encoder-decoder structural model based on the U-net model. The U-net model is one of the representative artificial neural network models developed for image segmentation in the biomedical field. The U-net model has a U-shaped process that rearranges various processes of the target process through a single neural network. The encoder-decoder structural model is an artificial intelligence model designed to separate and link input and output data processing to improve processing efficiency and quality. Each of the de-identification model and the plurality of artificial neural network models may be learned in the form of a ResNet-based imageNet pre-trained weight. mIoU (mean intersection over union) may be used as a learning reference, and Dice loss, Focal loss, etc. may be further used in addition to the Cross-Entropy loss commonly used.

The ResNET model is a multi-stage artificial neural network model of high performance developed by Microsoft. The issue of accuracy degradation that occurs when forming the multi-stage neural network to improve the performance is solved by utilizing a difference between input and output at each stage for learning, called residual learning. The imageNet is a large-scale database for developing a software that recognizes objects in the images, wherein the pre-trained weight refers to a condition of the initial artificial intelligence model learned to assign weights to a specific condition based on this data. The mIoU (mean Intersection over Union) is one of the methodologies for evaluating digital image analysis models, and is a method of quantifying a match rate of the results by dividing the number of common pixels between a target and a predicted result by the total number of pixels. The Cross-Entropy loss is one of the methodologies for evaluating digital analysis models, and uses an entropy-based loss function to evaluate a difference between probability distribution of target data and probability distribution of predicted model result data. The Focal loss is one of the methodologies for evaluating digital analysis models, and is an evaluation method that uses weighting parameters to focus on samples that are difficult to analyze by reducing the impact of easy-to-analyze sample data on overall prediction model learning.

Due to the nature of a skin, in most cases, the skin is in an environment (imbalanced data) with many general skin areas and few lesion areas. Therefore, for smooth learning, a learning method is used that measures the frequency of each label and reflects it when calculating loss. As the diagnostic model is separated for each item, individual characteristics are optimized and overlapping diagnosis for each item is possible. Further, by applying the de-identification model to a separate face image, security issues, for example, personal information protection for diagnostic information such as personal identification data that are difficult to remove with only the face detection model can be addressed.

FIG. 8 is a table that summarizes main items for diagnosing a skin by each of a plurality of artificial neural network models according to an embodiment of the present application.

Referring to FIG. 8, a wrinkle model for diagnosing wrinkles may be learned by adjusting parameters including the total number of a subject with detected wrinkles, an estimate of intensity (depth) compared to the surrounding undetected area in the subject with detected wrinkles, the total area with detected wrinkles, and the length and width of the detected wrinkles, and may output analysis results for the number of wrinkles, intensity (depth) of wrinkle, wrinkle area, wrinkle length, wrinkle width, distribution for each intensity, area, length or width of wrinkles, and wrinkle score.

A pigmentation model for diagnosing pigmentation may be learned by adjusting parameters including the total number of a subject with detected pigmentation, an estimate of intensity compared to the surrounding undetected area in the subject with detected pigmentation, the total area with detected pigmentation, and may output analysis results for the number of pigmentation, intensity of pigmentation, pigmentation area, distribution for each intensity or area of pigmentation, and pigmentation score.

A pore model for diagnosing pores may be learned by adjusting parameters including the total number of a subject with detected pores, an estimate of intensity (depth) compared to the surrounding undetected area in the subject with detected pores, the total area with detected pores, pore length and pore width, and may output analysis results for the number of pores, depth of pores, pore area, pore length, pore width, pore sagging (length to width ratio), distribution for each intensity, area, length, width or sagging of pores, and pore score.

An erythema model for diagnosing erythema may be learned by adjusting parameters including the total number of a subject with detected erythema, an estimate of intensity compared to the surrounding undetected area in the subject with detected erythema, the total area with detected erythema, and may output analysis results for the number of erythema, intensity of erythema, erythema area, distribution for each intensity or area of erythema, and erythema score.

An aging diagnosis model may output analysis results for facial aging or facial skin age estimated from a face image.

Further, the face detection model may output a facial size, which is a size of detected skin area, after de-identifying the face image.

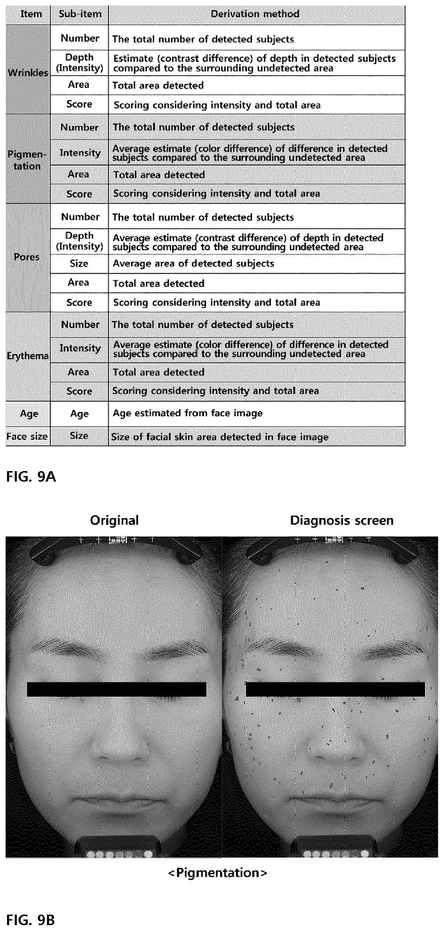

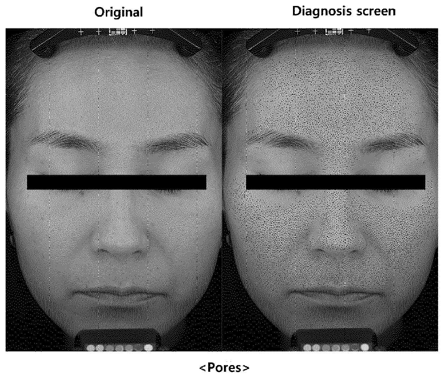

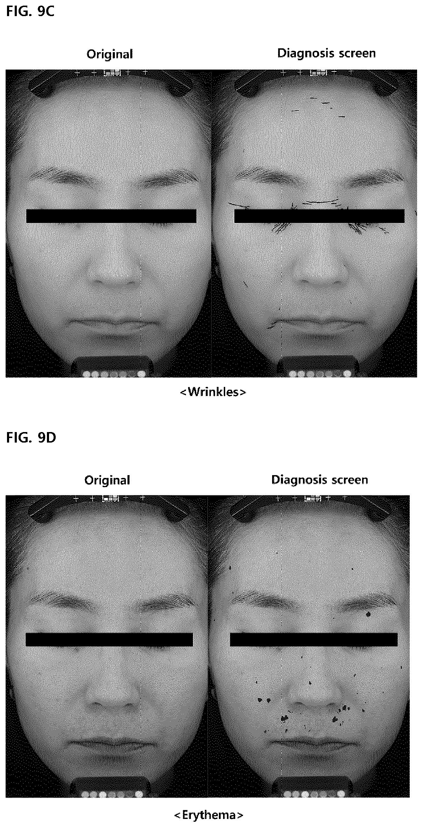

FIGS. 9A to 9D are images in which the diagnosis results for each item of de-identification models and a plurality of artificial intelligence neural network models are reconstructed into images before de-identification according to an embodiment of the present application.

Referring to FIGS. 9A to 9D, a skin diagnosis system 1 can diagnose and analyze each symptom in a skin image to quantitatively derive location and shape, and severity of each detailed subject with the actual skin condition symptom, and can perform analysis for the number, average size, shape, overall area, or intensity of the symptom, and can in turn provide visualization information in the skin image that emphasizes the diagnosis by utilizing the obtained information.

Further, the skin diagnosis system 1 separately consists of a de-identified model for processing individual information and a model for each diagnostic symptom including diagnoses for wrinkles, pigmentation, pores, and erythema, so that optimization and function separation or adjustment can be performed for each characteristic, which enables overlapping diagnosis and efficient system management and operation.

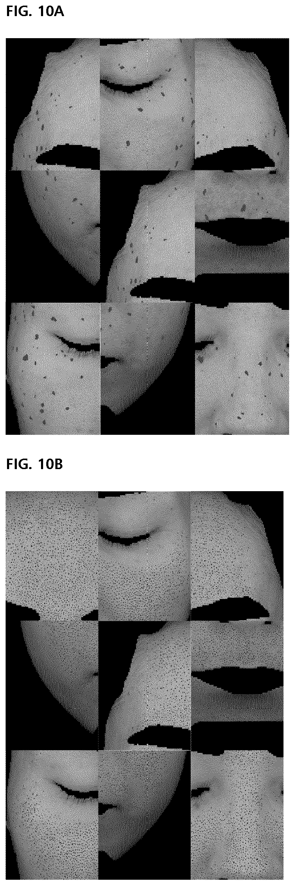

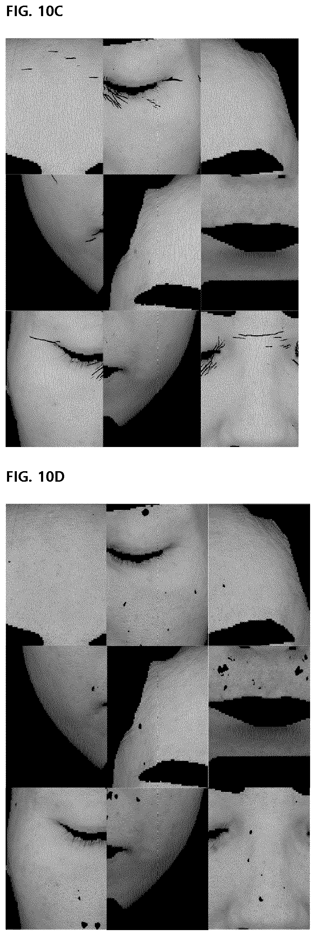

FIGS. 10A to 10D are diagnostic images for each item of a plurality of artificial intelligence neural network models when de-identification is performed using de-identification models according to an embodiment of the present application.

Each of FIGS. 10A to 10D is visualized images for the corresponding items using the pigmentation model, the pore model, the wrinkle model, and the erythema model, after de-identifying each original of FIGS. 9A to 9D with the de-identification model. Referring to FIGS. 10A to 10D, the de-identification of a face image may include: separating feature points and a background from the original face image and removing a skin shape having the feature points; dividing the face image from which the feature points have been removed into a plurality of patches; and rearranging the plurality of patches by randomizing them.

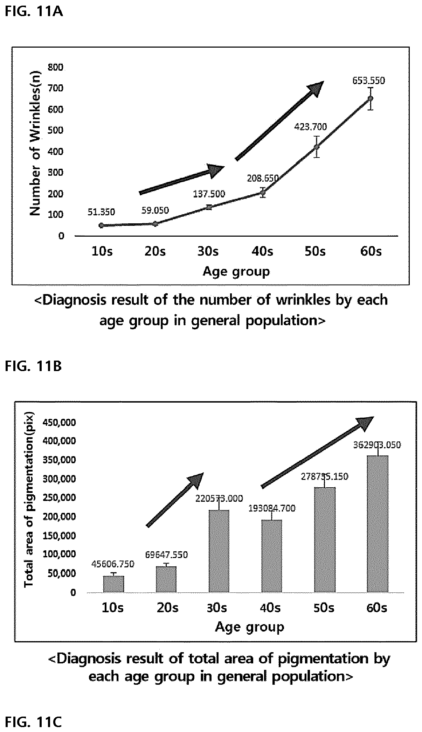

FIGS. 11A to 11D are graphs showing correlation between the diagnostic results for each skin diagnostic item as age increases, according to an embodiment of the present application.

Referring to FIGS. 11A to 11D, a diagnostic analysis of skin characteristics for each age was performed using facial skin images of various ages to check the consistency and analysis usability of the present application for Koreans having various ages and skin conditions and to analyze new skin characteristics and derive insights. In an experimental example, face images of a total of 120 Korean women including 20 women aged 14 to 69 by age group were used as subjects. Referring to FIG. 11A, as a result of analyzing the face images of 120 women in the normal skin group, the number of wrinkles all showed an increasing correlation with age, and overall, the occurrence of wrinkles appeared to accelerate at the change range from the 30s to 50s.

Referring to FIG. 11B, the total area of pigmentation showed an increasing correlation with age, and it seemed that the incidence of pigmentation increased significantly in the 30s, plateaued in the 40s, and then increased further in the 50s.

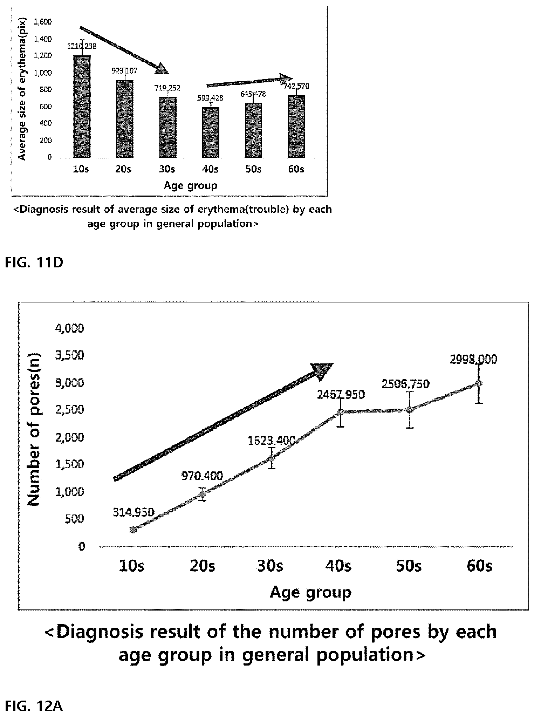

Referring to FIG. 11C, in case of erythema (trouble), the average size or the total area showed a correlation in which the average size or the total area decreased with age, and overall, it occurred at a high rate in low age groups such as those in their 10s and 20s, then decreased until their 30s and 40s, and then increased to some extent. It was judged that the temporary troubles of the young age had a greater effect than aging.

In case of pores, the number/average size/total area of pores showed an increasing correlation with age, while the intensity (depth) decreased. Referring to FIG. 11D, it showed that the number of pores increased overall, but a stagnation pattern in women in their 50s.

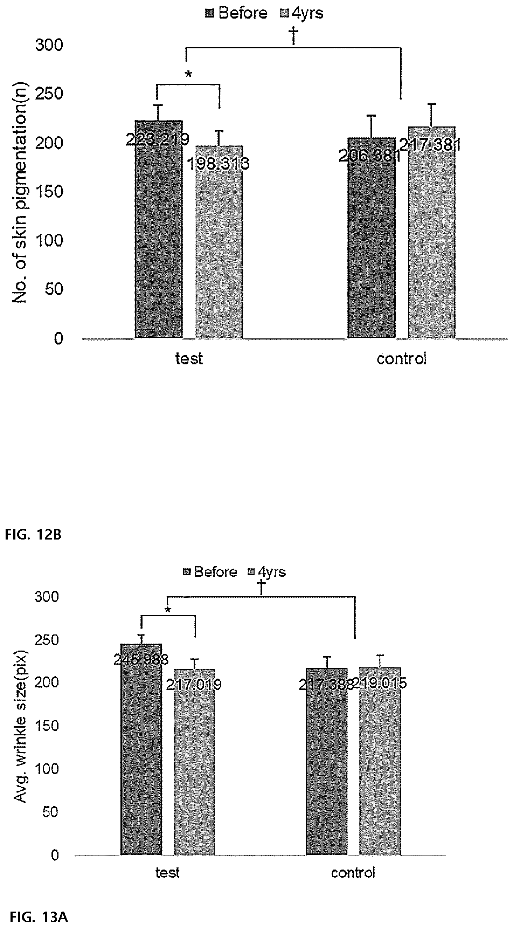

FIGS. 12A and 12B are graphs that analyze the results of an anti-aging product evaluation study according to an embodiment of the present application.

Referring to FIGS. 12A and 12B , the skin diagnosis system 1 may evaluate at least one of an antioxidant efficacy and a whitening efficacy for a specific product based on the skin diagnosis results for the above items. Unlike evaluation of an expert, the skin diagnosis system 1 can respond to subtle changes or large-scale repetitive follow-up observations using the same references, and can quantitatively evaluate and visualize various items using only the images, so that it can utilized for research on skin changes and evaluation of the efficacy of cosmetics and can be useful for customer service and creating more trustworthy customer communication content.

In an experimental example, as a result that the skin diagnosis was performed on images of long-term skin tracking research results of a retinol product that had proven anti-aging efficacy, it was confirmed during the 4-year follow-up period that the group that used the product showed a significant skin improvement effect, while the pigmentation and the number of wrinkles increased or remained in the group that did not use the product. This made it possible to provide more detailed and quantified evaluation results with the same results as separate clinical measurement evaluation results.

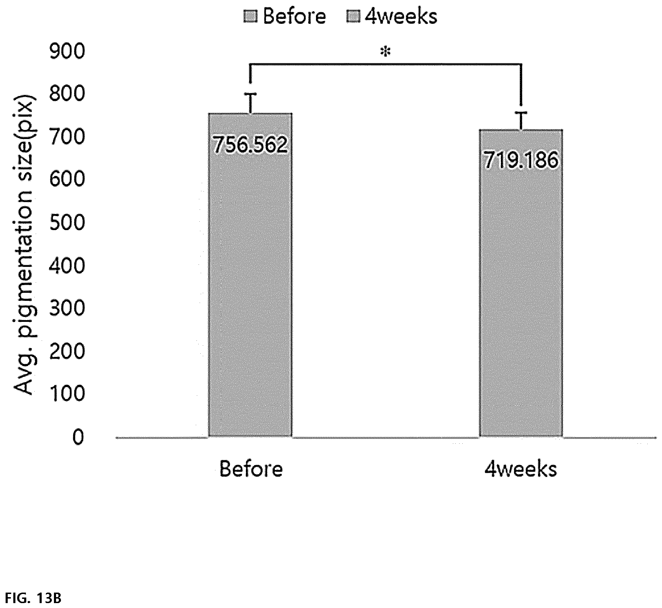

FIGS. 13A and 13B are graphs that analyze the results of a whitening product evaluation study according to an embodiment of the present application.

Referring to FIGS. 13A and 13B, as a result of applying the skin diagnosis system 1 to evaluate an efficacy of the whitening product, it was confirmed that a size and area of pigmentation in test participants who used a product containing 3,4,5-trimethoxycinnamatethymolester (TCTE) were significantly decreased after 4 weeks, which was consistent with the results of actual human application test. Therefore, it was judged that the skin diagnosis using deep learning could also be used appropriately for evaluating the efficacy of cosmetics, which requires evaluating a change in more subtle and various items than evaluating a pharmaceutical product, just by taking the image.

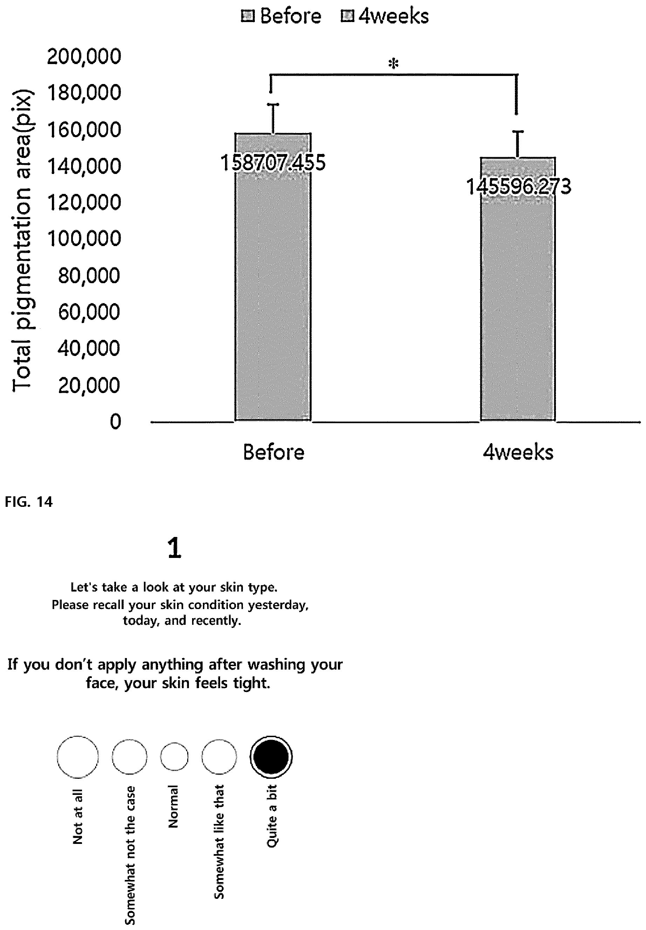

FIG. 14 shows a process for obtaining information about a subject's skin concerns and lifestyle through a questionnaire according to an embodiment of the present application.

Referring to FIG. 14, the skin diagnosis system 1, in addition to inputting de-identified face images into a plurality of artificial neural network models, respectively, can obtain information about the subject's skin concerns and lifestyle through a questionnaire. The skin diagnosis result may be the one related to the subject's skin concerns and lifestyle. The skin diagnosis system 1 can use information obtained through the questionnaire to provide diagnostic results by further focusing and specifying on the subject's skin concerns.

Based on the skin diagnosis results, the skin diagnosis system 1 may recommend a specific product tailored to the subject's skin concerns and lifestyle or provide beauty eating habits.

In an embodiment, the skin diagnosis system 1 provides analysis results of the subject's skin condition for each item of pigmentation, pores, erythema, and wrinkles. Further, information obtained through the questionnaire is used to provide comments on the subject's skin concerns and provide tips related to cleansing or lifestyle. In addition to a customized skin care method according to the subject's skin condition, cosmetics suitable for each skin condition can be recommended.

Since such a skin diagnosis system and method based on image analysis using deep learning are separately composed of the recognition model of facial location and feature points, the de-identification model of personal information, and a model for each diagnostic symptom including diagnoses for wrinkles, pigmentation, pores, erythema, and aging, optimization and function separation or adjustment can be performed for each characteristic, which enables overlapping diagnosis and efficient system management and operation.

The skin diagnosis can be performed even with a partial skin image that has been de-identified from the acquired face image. Since the diagnosis system is designed not to utilize, store, and/or process personal information, system operation and data collection can proactively respond to management without leaking the personal information.

The images used in the system to diagnose skin condition are not limited to images captured from a professional photography equipment, a general camera, or a smartphone, and are designed to derive the characteristics of each skin diagnosis item according to the image condition, so that the diagnosis system can be used for various purposes by minimizing the impact on a type or form of the image.

In long-term research on skin aging, such as anti-aging research, that requires long-term follow-up analysis, evaluation of the efficacy of anti-aging products, and evaluation of the efficacy of whitening cosmetics that require analysis of subtle change in skin pigment, evaluation of skin change and the efficacy of cosmetics can be quantitatively performed. Therefore, various skin diagnostic items can be performed quickly and at low cost without a separate skin measurement equipment or analysis preparation, thereby providing advantageous characteristics in the fields such as long-term follow-up or large-scale skin research.

The operation of the image analysis-based skin diagnosis system and method according to the embodiments described above may be at least partially implemented by a computer program and recorded on a computer-readable recording medium. For example, it may be implemented together with a program product consisting of the computer-readable medium having a program code, which can be executed by a processor to perform any or all steps, operations, or processes described.

The computer-readable recording medium includes all types of the recording devices for storing data that can be read by a computer. The computer-readable recording medium includes, for example, ROM, RAM, CD-ROM, a magnetic tape, a floppy disk, an optical data storage device, etc. Additionally, the computer-readable recording medium may be distributed across a computer system connected to a network, and may be stored and executed in a distributed manner by a computer-readable code. Further, a functional program, a code, and a code segment for implementing these embodiments can be easily understood by those skilled in the art to which these embodiments belong.

As described above, the present application is described with reference to the embodiments shown in the drawings, but these are merely illustrative examples, and those skilled in the art will understand that various modifications and variations of the embodiments can be carried out therefrom. However, such modifications should be considered to be within the technical protection scope of the present application. Therefore, the true technical protection scope of the present application should be determined by the technical spirit of the attached claims.

### [Industrial Applicability]

A skin diagnosis system and method based on image analysis using deep learning according to an aspect of the present application are separately of a face detection model that finds location of a face, a de-identification model that leaves only the skin area in a face image and performing random arrangement to de-identify personal information, and a model for each diagnostic symptom including diagnoses for wrinkles, pigmentation, pores, erythema, and aging. Therefore, optimization and function separation or adjustment can be performed for each characteristic, which enables overlapping diagnosis and efficient system management and operation.

## Claims

1. A skin diagnosis method based on image analysis using deep learning performed by a processor, the method comprising the steps of:
acquiring a face image of a subject by photographing a target skin;
deriving shape or location information of a facial structure by recognizing feature points capable of identifying an individual in the acquired face image;
de-identifying the face image on the basis of the shape or location information of the facial structure such that personal information of an analysis target cannot be identified; and
visualizing and providing skin diagnosis results for items corresponding to artificial neural network models and symptom locations for each item by inputting the de-identified face images into a plurality of the artificial neural network models , respectively,
wherein the items may include at least one of diagnoses for wrinkles, pigmentation, pores, erythema, and aging.

2. The skin diagnosis method according to claim 1,
wherein the step of de-identifying the face image includes:
separating feature points and a background from the face image and removing a skin shape having the feature points;
dividing the face image from which the feature points have been removed into a plurality of patches; and
rearranging the plurality of patches by randomizing them.

3. The skin diagnosis method according to claim 1,
wherein the feature points are at least one of eyebrows, eyes, nose, and lips.

4. The skin diagnosis method according to claim 1,
wherein the each of the plurality of artificial neural network models is learned using a plurality of training samples as learning data, and
the plurality of training samples include transformations using a data augmentation technique.

5. wherein the data augmentation technique includes at least one of random crop, blur, and flip processing.

6. The skin diagnosis method according to claim 1,
wherein the artificial neural network model for diagnosing the wrinkles is learned by adjusting parameters including the total number of a subject with detected wrinkles, an estimate of intensity (depth) compared to the surrounding undetected area in the subject with detected wrinkles, the total area with detected wrinkles, the length and width of the detected wrinkles, and
outputs analysis results for at least one among the number of wrinkles, intensity (depth) of wrinkles, wrinkle area, wrinkle length, wrinkle width, distribution for each intensity, area, length or width of wrinkles, and wrinkle score.

7. The skin diagnosis method according to claim 1,
wherein the artificial neural network model for diagnosing the pigmentation is learned by adjusting parameters including the total number of a subject with detected pigmentation, an estimate of intensity compared to the surrounding undetected area in the subject with detected pigmentation, the total area with detected pigmentation, and
outputs analysis results for at least one among the number of pigmentation, intensity of pigmentation, pigmentation area, distribution for each intensity, area, length or width of pigmentation, and pigmentation score.

8. The skin diagnosis method according to claim 1,
wherein the artificial neural network model for diagnosing the pores is learned by adjusting parameters including the total number of a subject with detected pores, an estimate of intensity (depth) compared to the surrounding undetected area in the subject with detected pores, the total area with detected pores, pore length and pore width, and
outputs analysis results for at least one among the number of pores, intensity (depth) of pores, pore size, pore area, pore length, pore width, pore sagging (length to width ratio), distribution for each intensity, area, length, width or sagging of pores, and pore score.

9. The skin diagnosis method according to claim 1,
wherein the artificial neural network model for diagnosing the erythema is learned by adjusting parameters including the total number of a subject with detected erythema, an estimate of intensity compared to the surrounding undetected area in the subject with detected erythema, the total area with detected erythema, and outputs analysis results for at least one among the number of erythema, intensity of erythema, erythema area, distribution for each intensity or area of erythema, and erythema score.

10. The skin diagnosis method according to claim 1,
wherein the artificial neural network model for diagnosing the aging predicts age for facial aging or facial skin aging estimated from the face image by inputting at least one of the de-identified face image, the output result of a single artificial neural network model, and the value that integrates the output result of a plurality of artificial neural network models.

11. The skin diagnosis method according to claim 1,
wherein each of the plurality of artificial neural network models is an encoder-decoder structural model based on U-net model.

12. The skin diagnosis method according to claim 1,
wherein each of the plurality of artificial neural network models is learned in the form of an imageNet pre-trained weight based on ResNet.

13. The skin diagnosis method according to claim 1,
further comprising the step of evaluating at least one of antioxidant efficacy and whitening efficacy for a specific product based on skin diagnosis results for the above items.

14. The skin diagnosis method according to claim 1,
further comprising the step of, before inputting the de-identified face image into the plurality of artificial neural network models, respectively, obtaining information about the subject's skin concerns and lifestyle through a questionnaire,
wherein the result of skin diagnose is the one resulting from the subject's skin concerns and lifestyle.

15. The skin diagnosis method according to claim 14,
further comprising the step of recommending a specific product tailored to the subject's skin concerns and lifestyle or providing beauty eating habit, based on the result of skin diagnose.

16. A skin diagnosis system based on image analysis using deep learning, the system comprising:
an imaging unit that acquires a face image by photographing a target skin;
a face detection model that derives shape or location information of a facial structure by recognizing feature points capable of identifying an individual in the acquired face image;
a de-identification model that de-identifies the face image on the basis of the shape or location information of the facial structure such that personal information of an analysis target cannot be identified; and
a plurality of artificial neural network models for each of at least one item among diagnoses for wrinkles, pigmentation, pores, erythema, and aging,
wherein the plurality of artificial neural network models receive the de-identified face image as input, and visualize and provide skin diagnosis results for items corresponding to the artificial neural network models and symptom locations for each item.
